# EUROPEAN PATENT APPLICATION

(11) **EP 4 821 385 A1**
(43) Date of publication of application: **14.10.2026**
(21) Application number: 24900684.2
(22) Date of filing: 05.12.2024
(51) Int. Cl.: A41D 31/00, A41B 11/00, A41D 13/06, A41D 13/08, A41D 19/00, A41D 31/18, A61F 13/06, A61F 13/08, A61F 13/10, D04B 1/00, D04B 1/18

(54) **ELASTIC GARMENT AND METHOD FOR PRODUCING SAME**

(30) Priority: 05.12.2023 JP 2023205685; 05.12.2023 JP 2023205686
(71) Applicant: Mitsui Medical Japan Co., Ltd., Tokyo 104-0061 (JP)
(72) Inventor: MITSUI Keiko, Tokyo 104-0061 (JP)
(74) Representative: Hoffmann Eitle
(86) International application number: PCT/JP2024/043133
(87) International publication number: WO 2025/121391

(57) **Abstract**

Provided is an elastic garment that can apply necessary compression and fits well to the region it is worn on, while being highly breathable and being easily gripped by the fingers to facilitate an action of pulling up in a wearing direction. The elastic garment is formed from a knitted fabric 10 in which a plain knit structure 20 knitted using a ground elastic yarn 21 and a thread knit structure 30 knitted using the ground elastic yarn 21 and a thread elastic yarn 31 that is thicker and relatively higher in stretch modulus than the ground elastic yarn 21 are alternately knitted.

## Description

### Technical Field

The present invention relates to an elastic garment formed from a knitted fabric including elastic yarns and a method for producing the same.

### Background Art

Lymph nodes are excised for the prevention of metastasis during cancer surgery and for other reasons. The excision of lymph nodes worsens lymph flow and causes the lymph to pool in the limbs, causing lymphedema. For the prevention and treatment of lymphedema, moderate exercise, lymph drainage, compression therapy, lymphovenous anastomosis, and so on are effective, and above all, compression therapy of wearing elastic stockings that apply strong compressive force is convenient and easy to implement in daily life.

As such an elastic stocking, Patent Document 1 discloses a stocking for medical use configured such that compressive forces applied to an ankle region, a lower leg region, and a thigh region are gradually reduced. This enhances the effect of improving lymph flow in the leg. In addition, a measure is taken to increase breathability and moisture permeability by setting the length ratio of a ground yarn and an insert yarn (elastic yarn) to a predetermined relationship.

Further, for the treatment of lymphedema and venous return disorder, Patent Document 2 proposes a cylindrical bandage adapted to changes in the cross-section of a flexed part of a human body's region on which it is worn. It discloses that the vertical cross-sectional dimensions at predetermined positions of the human body are measured, and a cylindrical bandage having corresponding cross-sectional dimensions is formed. Patent Documents 3 and 4 disclose compression garments of a sleeve type and a mitt type, which can be worn on an arm, a hand, and the like, instead of a lower limb.

Further, Patent Document 5 discloses a glove that corrects extension disorder occurring in a wrist joint and a finger joint. This glove includes stretchable yarns, and its palm portion is formed by inlay knitting, and its hand back portion is formed by short-row knitting or by inlay knitting including tuck knitting. This configuration applies a force that causes the hand to warp from the palm side toward the back of the hand. It is indicated that wearing this glove allows for the strengthening of muscle strength in the fingers and is effective for patients with Parkinson's disease.

### Prior Art Document

### Patent Document

Patent Document 1: Japanese Laid-open Patent Publication No. 2011-115571
Patent Document 2: Japanese Patent No. 6052753
Patent Document 3: Japanese Translation of PCT International Application Publication No. 2019-507833
Patent Document 4: Japanese Translation of PCT International Application Publication No. H11-5018228
Patent Document 5: Japanese Patent No. 7045029

### Summary of the Invention

### Problems to Be Solved by the Invention

The stocking for medical use of Patent Document 1 is circular-knitted, and an elastic yarn is inserted into a ground yarn knit structure by tucking. Since the elastic yarn inserted by tucking is not knitted into the ground yarn knit structure, there is a drawback in that the elastic yarn has difficulty in stabilizing in position and moves easily. If the position of the elastic yarn is unstable, the compression may not be exerted as designed.

In the cylindrical bandage of Patent Document 2, a base yarn forms a rib stitch structure by alternating knit stitches and purl stitches, and an elastic yarn repeats knitting and missing. Specifically, as illustrated in FIGs. 13 of Patent Document 2, the elastic yarn has no knitting relationship with the base yarn and is in a miss state with respect to the base yarn from beginning to end. Where the rubber yarn is missed, the base yarn is knitted in purl stitches. Therefore, as illustrated in the drawings of "a state in which external force is applied" in FIGs. 4 and FIGs. 5 of Patent Document 2, the elastic yarn forms a ladder-like float in the lateral direction and is not knitted into the base yarn. Consequently, when the elastic yarn contracts in the lateral direction, the knitted fabric becomes considerably thick and thus is stuffy during wear, leaving room for improvement in terms of breathability.

Furthermore, because of the rib knitting, the irregularities of the elastic yarn are unlikely to appear on the surface. Furthermore, a plurality of outer protrusions and a plurality of inner protrusions, of the base yarn, which extend continuously in the longitudinal direction of the cylindrical bandage are arranged in the circumferential direction. Thus, the outer protrusions and the inner protrusions extend in the vertical direction, and for example, when one inserts the toes into the bandage and tries to pull it up toward the thigh to wear it on the leg, the fingertips of the hand performing the action do not easily catch on the fabric and do not easily pull it up. The higher the compression, the more difficult it becomes to pull up. Among lymphedema patients, women have weak strength in their fingertips, and this is even more pronounced in the case of elderly patients.

Furthermore, the stocking for medical use of Patent Document 1 is basically formed in the same shape as an ordinary stocking. As is seen from the joint covering portion that covers the area around a joint, for example, at the ankle joint, the heel side bulges outward as illustrated in the drawings of Patent Document 1, and thus a portion to cover the heel is provided, but no special measure is taken for the method of knitting the inner side of the ankle joint. Thus, the number of stitches in the course direction and the number of stitches in the wale direction (the numbers of courses) are those required to form the inner side of the ankle joint. For this reason, the fabric tends to slack and wrinkle on the inner side of the ankle joint, making it difficult to apply pressure in the compression direction. Moreover, there is a possibility that the slack and wrinkles of the fabric may dig into the skin to obstruct lymph flow and blood flow. The same applies to the cylindrical bandage of Patent Document 2, and the same thing as in Patent Document 1 can be said regarding the illustrated portion covering the ankle joint. In Patent Document 2, however, it is formed after the circumferential lengths near the ankle joint are measured, from which it is considered that the fit is higher and slack or wrinkles on the inner side of the ankle joint are less likely to occur, compared to Patent Document 1. However, in Patent Document 2, it is necessary to measure the circumferential lengths of the worn region for the adaptation to changes in the cross-section, leading to a complicated and troublesome manufacturing process.

Furthermore, not only the stocking-type garments worn on the lower limb as in Patent Documents 1 and 2 but also elastic garments that can be used as countermeasures against lymphedema in arms and hands as in Patent Documents 3 and 4 are desired. Furthermore, it would be convenient if an elastic garment useful for strengthening and improving muscle strength of patients with Parkinson's disease and other such persons, as shown in Patent Document 5, can be manufactured with a similar structure.

The present invention has been made in consideration of the above, and has an object to provide an elastic garment that can apply a necessary compression and has excellent fit to a region it is worn on, while having high breathability, being easy to grip with fingers to facilitate an action of pulling it up in the wearing direction, and being suitable for improving or preventing lymphedema or venous return disorder. Another object is to provide an elastic garment that is less likely to fail in applying a necessary pressure or obstruct lymph flow and blood flow due to the occurrence of slack or wrinkles in a joint covering portion, is applicable to not only lower limbs but also arms, hands, and the like, and is suitable for improving or preventing lymphedema or venous return disorder, and to provide a method for producing the same. Another object is to provide an elastic garment that has a similar structure and can be utilized for improving muscle strength, and a method for producing the same.

### Means for Solving the Problems

To solve the above objects, the present invention provides an elastic garment that is knitted using at least two types of elastic yarns different in stretchability, the elastic garment being formed from a knitted fabric in which a plain knit structure and a thread knit structure are alternately knitted,
the plain knit structure being knitted using a ground elastic yarn, and
the thread knit structure being knitted using the ground elastic yarn and a thread elastic yarn that is thicker and higher in stretch modulus than the ground elastic yarn.

Preferably, the ground elastic yarn and the thread elastic yarn which constitute the thread knit structure are knitted such that knit and miss positions of the ground elastic yarn and the thread elastic yarn alternate stitch by stitch in a course directi on.

Further, preferably, the plain knit structure and the thread knit structure are knitted to alternate in a wale direction.

Preferably, core yarns of both the ground elastic yarn and the thread elastic yarn are polyurethane yarns, and
the polyurethane yarn constituting the ground elastic yarn has a thickness of 150 decitex or less, and the polyurethane yarn constituting the thread elastic yarn has a thickness of 550 decitex or more.

Preferably, the thickness of the polyurethane yarn constituting the ground elastic yarn is within a range of 30 to 100 decitex, and the thickness of the polyurethane yarn constituting the thread elastic yarn is within a range of 1000 to 2500 decitex.

Preferably, the knitted fabric is composed of only either a knit structure or a purl structure.

Further, the elastic garment is suitable for improving or preventing lymphedema or venous return disorder.

Further, preferably, the elastic garment includes
a tubular-shaped joint covering portion having an inner covering portion that covers a flexion-direction inner side of a joint and an outer covering portion that covers a flexion-direction outer side of the joint, and
a knitted fabric part of one of the inner covering portion and the outer covering portion in the joint covering portion at least partly has a course-reduced portion that has a smaller number of stitch courses in a wale direction than a knitted fabric part forming the other.

The elastic garment can be configured such that the course-reduced portion is provided in at least part of the knitted fabric part of the inner covering portion, and this elastic garment is suitable especially for improving or preventing lymphedema or venous return disorder.

The elastic garment can be configured such that the course-reduced portion is provided in at least part of the knitted fabric part of the outer covering portion, and this elastic garment is suitable especially for improving muscle strength.

Further, the elastic garment of the present invention can be configured as:
lower-body wear that has, as the joint covering portion, a portion covering at least one of a toe joint, an ankle joint, and a knee joint;
upper-body wear that has, as the joint covering portion, a portion covering at least one of a wrist joint, an elbow joint, a shoulder joint, and a finger joint; or
full-body wear in which the lower-body wear and the upper-body wear are integrated.

The present invention further provides a method for producing an elastic garment that is knitted using an elastic yarn and includes a tubular-shaped joint covering portion having: an inner covering portion covering a flexion-direction inner side of a joint; and an outer covering portion covering a flexion-direction outer side of the joint, the method including,
when knitting the joint covering portion,
making the number of stitch courses in a wale direction in at least part of a knitted fabric part of one of the inner covering portion and the outer covering portion smaller than in a knitted fabric part forming the other.

In this case, it is possible to provide a method for producing an elastic garment for improving or preventing lymphedema or venous return disorder, by making the number of courses in at least part of the knitted fabric part of the inner covering portion smaller than in the knitted fabric part forming the outer covering portion.

Further, it is possible to provide a method for producing an elastic garment for improving muscle strength, by making the number of courses in at least part of the knitted fabric part of the outer covering portion smaller than in the knitted fabric part forming the inner covering portion.

### Effect of the Invention

The elastic garment of the present invention is formed from a knitted fabric in which a plain knit structure and a thread knit structure are alternately knitted, the plain knit structure being knitted using a ground elastic yarn, and the thread knit structure being knitted using the ground elastic yarn and a thread elastic yarn that is thicker and relatively higher in stretch modulus than the ground elastic yarn. Since the thread elastic yarn is thicker than the ground elastic yarn, on one surface side of the thread knit structure, the thread elastic yarn protrudes slightly more in the thickness direction than the ground elastic yarn. This creates slight steps between a knitted fabric of the thread knit structure and a knitted fabric of the plain knit structure, resulting in a bellows-like shape. When the garment is gripped with fingers, the fingers easily catch on the steps, facilitating the pulling-up action and overcoming wearing difficulty that is characteristic of elastic garments. Further, since the thread elastic yarn is interconnected with the ground elastic yarn by knitting, this method can more stably arrange the thread elastic yarn and cause a more appropriate compression to be exerted as designed than a method where an elastic yarn is inserted by tucking or inlaying. Furthermore, because of the abovementioned bellows-like shape having the slight steps, the garment easily fits the region it is worn on, and even at a joint region, it expands and contracts following flexion movements, making slack and wrinkles unlikely to occur on the flexion-direction inner side. Since slack and wrinkles are unlikely to occur, compression is appropriately exerted on this region, and the obstruction of lymph flow and blood flow due to slack or wrinkles is also reduced.

In addition, as the ground elastic yarn, a thin yarn of a predetermined size or less is used to form the plain knit structure, and preferably, the knitted fabric is knitted with only either a knit structure or a purl structure, thereby achieving high breathability.

Furthermore, preferably, in a joint covering portion that covers the area around a joint, a knitted fabric part of one of an inner covering portion, which covers a flexion-direction inner side of the joint, and an outer covering portion, which covers a flexion-direction outer side of the joint, at least partly has a course-reduced portion that has a smaller number of stitch courses in a wale direction than a knitted fabric part forming the other. Consequently, in the joint covering portion, a relatively strong tension acts on the knitted fabric with fewer courses during wear. Therefore, providing the knitted fabric part with fewer courses in the inner covering portion makes slack and wrinkles less likely to occur on the inner side when the joint is flexed, allowing this region to be compressed with a predetermined pressure. Moreover, obstruction of lymph flow and blood flow due to slack or wrinkles can be reduced. On the other hand, providing the knitted fabric part with fewer courses in the outer covering portion causes a relatively strong tension to act in the outer covering portion, which acts as resistance when the joint is flexed, and can contribute to the strengthening and improvement of muscle strength.

### Brief Description of Drawings

[FIG. 1] FIG. 1 is a perspective view of an elastic stocking, which is an elastic garment according to a first embodiment of the present invention, as viewed from one side.
[FIG. 2] FIG. 2 is a perspective view of the elastic stocking in FIG. 1 in a reversed state, as viewed from the opposite side.
[FIG. 3] FIG. 3 is a partially enlarged view of FIG. 1.
[FIGs. 4] FIG. 4(a) is a view schematically illustrating a structure knitted with a ground elastic yarn and a thread elastic yarn, and FIG. 4(b) is a view illustrating knit and miss stitches using symbols.
[FIGs. 5] FIGs. 5 are views illustrating part of a stocking, which is an elastic garment according to a second embodiment of the present invention, in a state in which the elastic garment is worn on a leg, (a) being a view seen from the side of the leg, and (b) being a view seen from the back of the knee.
[FIGs. 6] FIG. 6(a) is a view along arrow A in FIG. 5(a), FIG. 6(b) is a view along arrow B in FIG. 5(a), and FIG. 6(c) is a view along arrow C in FIG. 5(b).
[FIG. 7] FIG. 7 is a view for explaining a method of knitting a course-reduced portion.
[FIGs. 8] FIGs. 8 are views illustrating an example of a stocking in which a step-reduced portion is provided in a joint covering portion for an ankle joint, (a) being a side view of the stocking, and (b) being a view illustrating a state in which the stocking in (a) is worn on an ankle joint.
[FIG. 9] FIG. 9 is a view illustrating a glove, which is an elastic garment according to a third embodiment of the present invention.
[FIG. 10] FIG. 10 is a view along arrow D in FIG. 9.

### Modes for Carrying out the Invention

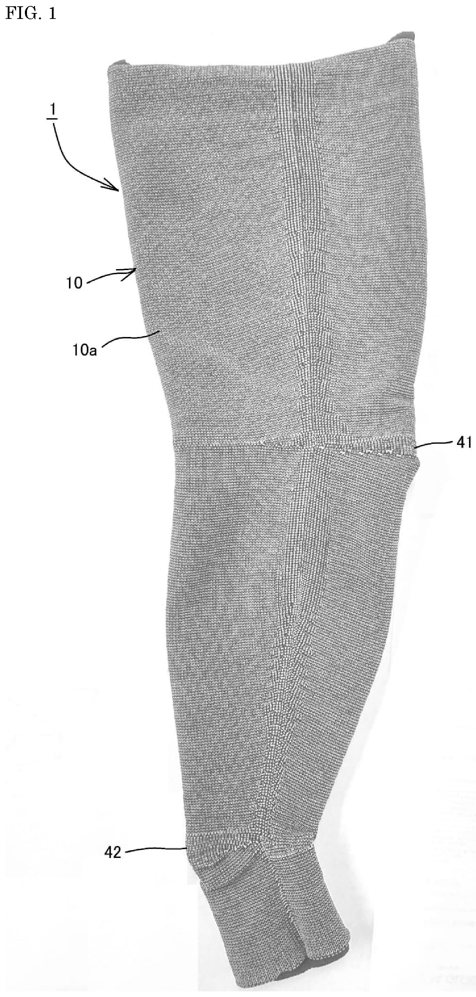

Embodiments of the present invention will be hereinafter described with reference to the drawings. FIG. 1 to FIGs. 4 are views for explaining an elastic stocking 1 as an elastic garment according to a first embodiment of the present invention.

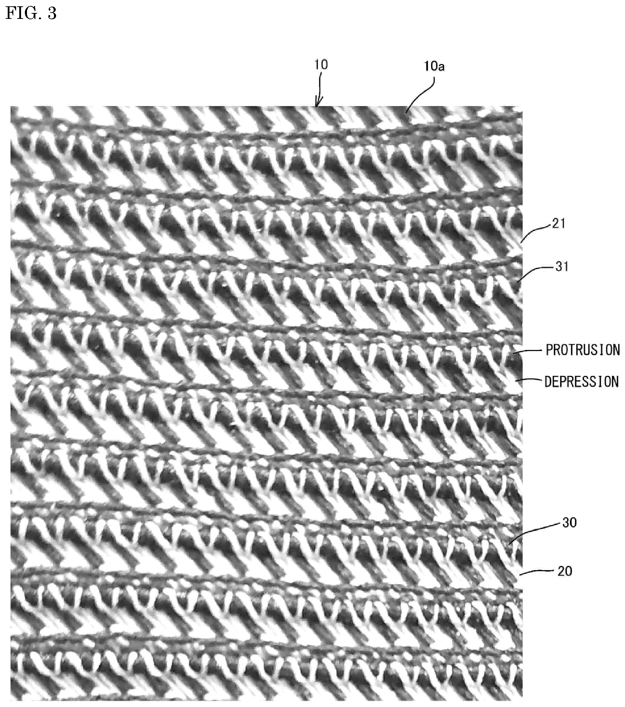

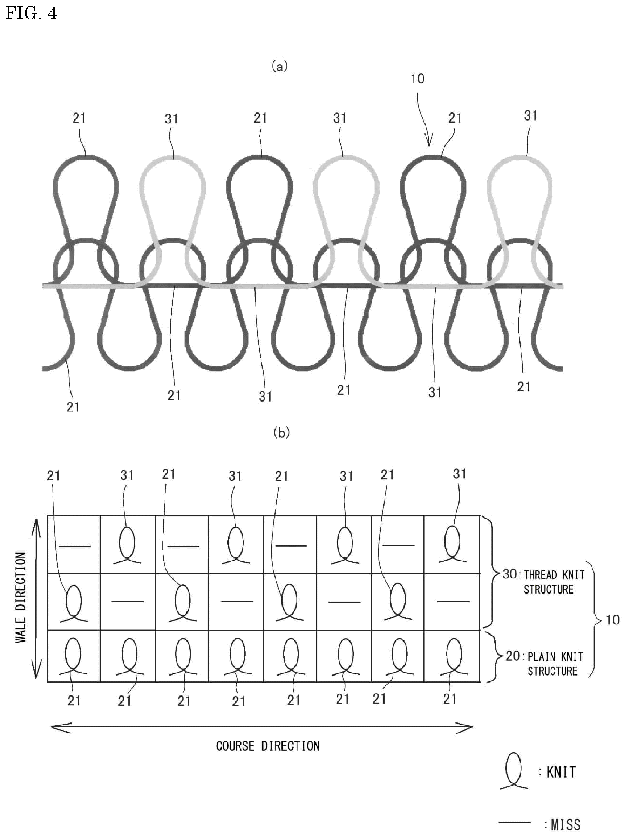

A knitted fabric 10 constituting the elastic stocking 1 of this embodiment is knitted using at least two types of elastic yarns that are different in stretchability. Specifically, as illustrated in FIG. 3 and FIGs. 4, it is knitted using a ground elastic yarn 21, which serves as a ground yarn, and a thread elastic yarn 31.

A plain knit structure 20 is formed by weft knitting using the ground elastic yarn 21, and a thread knit structure 30 is formed by weft knitting using the ground elastic yarn 21 and the thread elastic yarn 31. The knitted fabric 10 of this embodiment is configured such that the plain knit structures 20 and the thread knit structures 30 alternate in the wale direction.

Both the ground elastic yarn 21 and the thread elastic yarn 31 are preferably covered yarns, and as their core yarns, rubber yarns, polyurethane yarns, or the like are usable. When the ground elastic yarn 21 and the thread elastic yarn 31 are compared in terms of stretch modulus, a yarn having a relatively low stretch modulus is used as the ground elastic yarn 21, while a yarn having a relatively high stretch modulus is used as the thread elastic yarn 31. In the case where they both use polyurethane yarns as their core yarns, stretch modulus is lower in a thinner one and is higher in a thicker one.

In the case where they both use the polyurethane yarns as their core yarns, it is preferable that the ground elastic yarn 21 uses a polyurethane yarn having a thickness of 150 decitex or less, and the thread elastic yarn 31 uses a polyurethane yarn having a thickness of 550 decitex or more. The thickness of the polyurethane yarn constituting the ground elastic yarn 21 is more preferably within a range of 30 to 100 decitex, and still more preferably within a range of 40 to 80 decitex. The thickness of the polyurethane yarn constituting the thread elastic yarn 31 is more preferably within a range of 1000 to 2500 decitex, and still more preferably within a range of 1500 to 2300 decitex. If the thickness of the polyurethane yarn exceeds 2500 decitex, knitting with a weft knitting machine becomes difficult.

Therefore, the thickness relationship between the polyurethane yarn constituting the ground elastic yarn 21 and the polyurethane yarn constituting the thread elastic yarn 31 is within a range of 1:3.6 to 1:83.3, where the thickness of the former is defined as 1. As a result, the ground elastic yarn 21 has a thickness of about 1/3 or less of that of the thread elastic yarn 31, and the plain knit structure 20 knitted with the ground elastic yarn 21 is reduced in thickness, ensuring high breathability.

FIG. 4(a) is a view illustrating stitches of the knitted fabric of this embodiment, and FIG. 4(b) is a schematic view illustrating the stitches denoted by symbols. As illustrated in these drawings, the plain knit structure 20 constituted by the ground elastic yarn 21 is knitted in the course direction, and in the next row in terms of the wale direction, the thread knit structure 30 constituted by the ground elastic yarn 21 and the thread elastic yarn 31 is knitted. These knit structures as one unit are composed of only either a knit structure or a purl structure and are repeated in the wale direction, whereby the knitted fabric is formed in which the plain knit structures 20 and the thread knit structures 30 alternate.

In the thread knit structure 30, the thread elastic yarn 31 is knitted such that its knit and miss positional relations with respect to the ground elastic yarn 21 alternate stitch by stitch in the course direction such as "knit, miss, knit, miss...". In this embodiment, since the thread elastic yarn 31 is knitted into the ground elastic yarn 21 rather than being tucked or inlaid, the arrangement position of the thread elastic yarn 31 is stabilized, and it moves less during wear.

In addition, the elastic stocking 1 has a knee-corresponding knitted fabric 41, which is three-dimensionally knitted so as to protrude slightly forward in front of the knee joint region, and a heel-corresponding knitted fabric 42, which is three-dimensionally knitted so as to protrude backward at a flexion-direction outer side of the ankle joint region. These portions are knitted together with the abovementioned plain knit structures 20 or thread knit structures 30.

According to this embodiment, the plain knit structures 20 and the thread knit structures 30 are formed so as to alternate in the wale direction using the two types of elastic yarns different in thickness, namely, the ground elastic yarn 21 using the polyurethane yarn that is relatively thin and low in stretch modulus, and the thread elastic yarn 31 using the polyurethane yarn that is relatively thick and high in stretch modulus. Furthermore, as illustrated in FIG. 4(b), since the thread elastic yarn 31 is knitted in a "knit, miss, knit, miss..." pattern along the course direction, the thick thread elastic yarn 31 protrudes outward by its thickness on one surface of the thread knit structures 30. Since the thread elastic yarn 31 is arranged in the course direction, that is, in the circumferential direction of the elastic stocking 1, steps formed by protrusions centered on the thread elastic yarn 31 and depressions near the plain knit structures 20 are formed in the circumferential direction on one surface of the knitted fabric 10, as illustrated in FIG. 3, resulting in a so-called bellows-like shape. Accordingly, when the wearer tries to wear the elastic stocking 1 of this embodiment by inserting his/her toes and pulling up the knitted fabric toward the calf and thigh via the heel by gripping it with fingers, the fingers easily catch on the steps formed so as to be substantially perpendicular to the pulling direction. Thus, the elastic stocking 1 of this embodiment is easier to wear than the elastic stockings of Patent Documents 1 and 2.

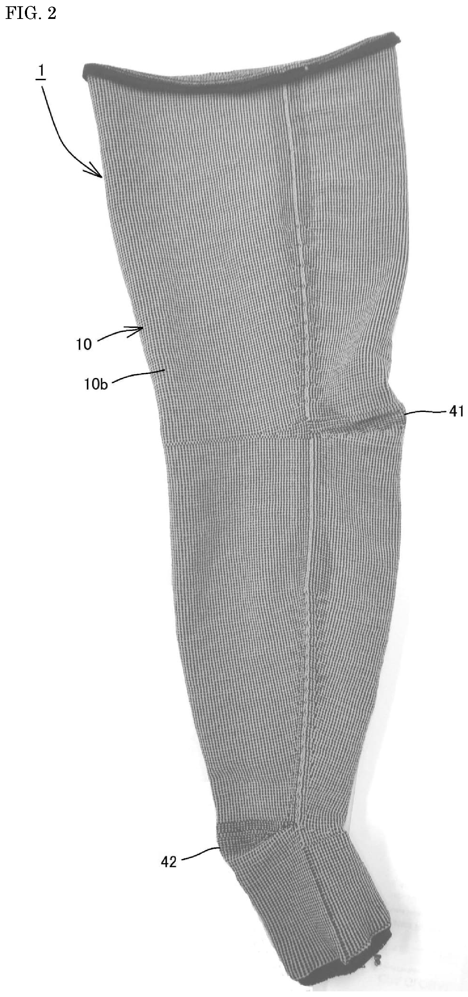

FIG. 1 is a perspective view in which a surface (stepped surface) 10a on which the steps formed by the depressions and the protrusions appear along the circumferential direction is on the outer side. When the wearer tries to wear it in the state illustrated in FIG. 1, the index finger, middle finger, and so on other than the thumb easily catch on the steps. In contrast to the stepped surface 10a, a surface on the inner side thereof is a substantially flat surface (flat surface) 10b because, on the flat surface 10b, the thread elastic yarn 31 does not protrude in the thickness direction. FIG. 2 is a perspective view illustrating a state reversed from the state in FIG. 1, that is, a state in which the stepped surface 10a where the depressions and the protrusions appear is on the inner side, and the flat surface 10b is on the outer side. In this case, since the stepped surface 10a is on the inner side, the thumb easily catches on the steps at the time of wearing. Therefore, regardless of whichever surface is on the outer side, the elastic stocking 1 of this embodiment can be easily pulled up at the time of wearing. Therefore, either the stepped surface 10a or the flat surface 10b can be on the outer side, and by wearing it reversibly, one can also enjoy design variation.

However, when the stepped surface 10a is on the outer side and the flat surface 10b is on the inner side, since a surface in contact with the skin is the flat surface 10b, it gives little sense of discomfort upon contact. There are cases where skin symptoms such as cellulitis appear due to lymphedema, and in such cases, the flat surface 10b, which causes less discomfort, is preferably on the inner side. On the other hand, when the stepped surface 10a is on the inner side, contact friction with the skin increases, resulting in a higher effect of inhibiting displacement during wear. Therefore, if no major skin disease is present, it is also effective to wear it with the stepped surface 10a on the inner side to inhibit displacement.

Furthermore, since the stretch modulus of the thread elastic yarn 31 is high, the ground elastic yarn 21 constituting the plain knit structures 20 stretches relatively greater than the thread elastic yarn 31 during wear. As a result, gaps between the ground elastic yarns 21 forming the plain knit structures 20 tend to become larger in the wearing direction. Moreover, the fact that the knitted fabric is composed of only either the knit structure or the purl structure also promotes easy stretching of the ranges of the plain knit structures 20. For these reasons, the elastic stocking 1 of this embodiment exhibits high breathability.

On the other hand, in the ranges of the thread knit structures 30, a high contractive force of the thread elastic yarn 31 having a high stretch modulus acts. Moreover, since the thread elastic yarn 31 is incorporated by knitting, a certain compression can be applied to a predetermined human body's region in contact. Generally, regarding the compression by an elastic yarn, a higher compression is obtained when the elastic yarn expands and contracts linearly than when it deforms into a loop shape. In view of this point, conventionally, an elastic yarn is often inserted by inlaying or tucking. In contrast, according to this embodiment, the thread elastic yarn 31 has the abovementioned difference in thickness from the ground elastic yarn 21, and during wear, the ground elastic yarn 21 stretches preferentially over the thread elastic yarn 31, while the thread elastic yarn 31 hardly changes into a loop shape even though it is knitted, but changes into a substantially linear shape. This ensures that certain compression is exerted.

In addition, due to the high stretchability of the plain knit structures 20 and the high elasticity of the thread knit structures 30, slack and wrinkles are unlikely to occur on the inner side of a flexed part such as a knee joint region and an ankle joint region. This enhances the fit, so that the obstruction of lymph flow and blood flow due to slack and wrinkles is less likely to occur.

Possessing such characteristics, the elastic stocking 1 of this embodiment can be worn more easily even by women and elderly people with weak strength in their fingers. Furthermore, the high breathability reduces stuffiness, allowing for comfortable wearing for a longer period, which is effective for improving lymphedema, venous return disorder, and the like. In addition, the ground elastic yarn 21 stretches by a certain amount depending on the thickness and length of the leg, and if the stretching exceeds a predetermined level, the thread elastic yarn 31 also stretches further. Therefore, because of the very stretchable structure, the elastic stocking 1 has little dependence on the thickness or length of the leg and can be substantially one-size-fits-all.

In this embodiment, the elastic stocking 1 is described as an example, but the invention is not limited thereto. Stockings are ordinarily those having welt portions, leg portions, and foot portions and extending above the knees, and are included in the concept of "socks". However, this embodiment is also applicable to other forms of "socks", namely, foot covers (products with only a foot portion), socks (products having a top rubber portion, a leg portion, and a foot portion and reaching below the knee), tights (products with a panty portion, leg portions, and foot portions), and further to those with a structure having no foot portions, such as leggings and compression shorts.

Besides, the configuration of this embodiment is also applicable to a sleeve having a portion that covers at least one of a wrist joint, an elbow joint, and a shoulder joint where lymphedema or the like is likely to occur, a glove having a portion that covers at least one of a wrist joint and finger joints, a garment in which a sleeve or a glove is integrally provided, or the like. It is also applicable to a bodysuit or the like in which wear worn on the lower body, such as stockings, and wear worn on the upper body are integrated. In these cases as well, whether the stepped surface 10a or the flat surface 10b is to be on the outer side can be determined in consideration of skin symptoms, ease of displacement, and the like. For example, in the case of a sleeve worn on a range from the arm to the armpit, since it is relatively easy to slide down, it is preferably worn with the stepped surface 10a on the inner side if no skin symptoms have appeared.

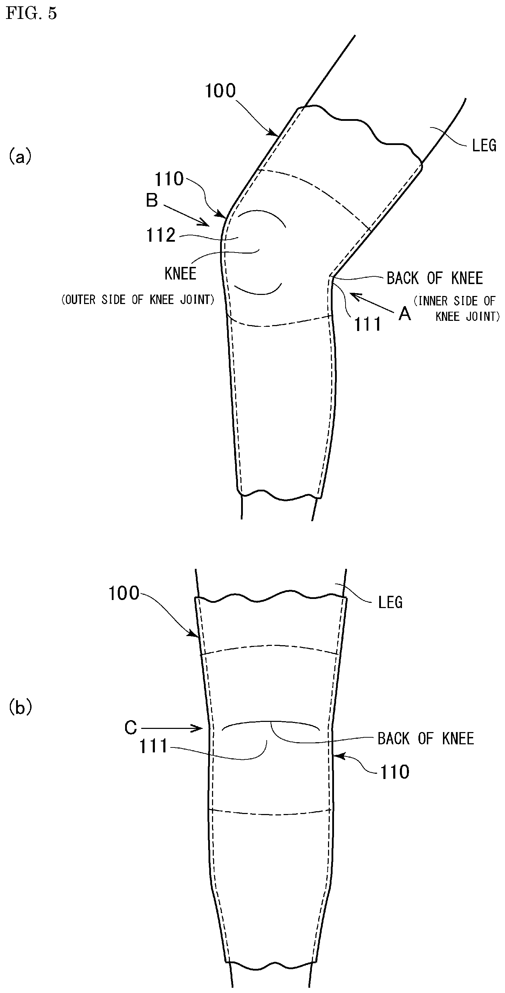

Next, second and third embodiments of the present invention will be described based on FIGs. 5 to FIG. 10. As in the first embodiment, in elastic garments of the second and third embodiments, a knitted fabric is knitted using a ground elastic yarn 21, which serves as a ground yarn, and a thread elastic yarn 31. Further, a plain knit structure 20 is formed by weft knitting using the ground elastic yarn 21, and a thread knit structure 30 is formed by weft knitting using the ground elastic yarn 21 and the thread elastic yarn 31. The knitted fabric of each of the embodiments is configured such that the plain knit structures 20 and the thread knit structures 30 are knitted so as to alternate in the wale direction.

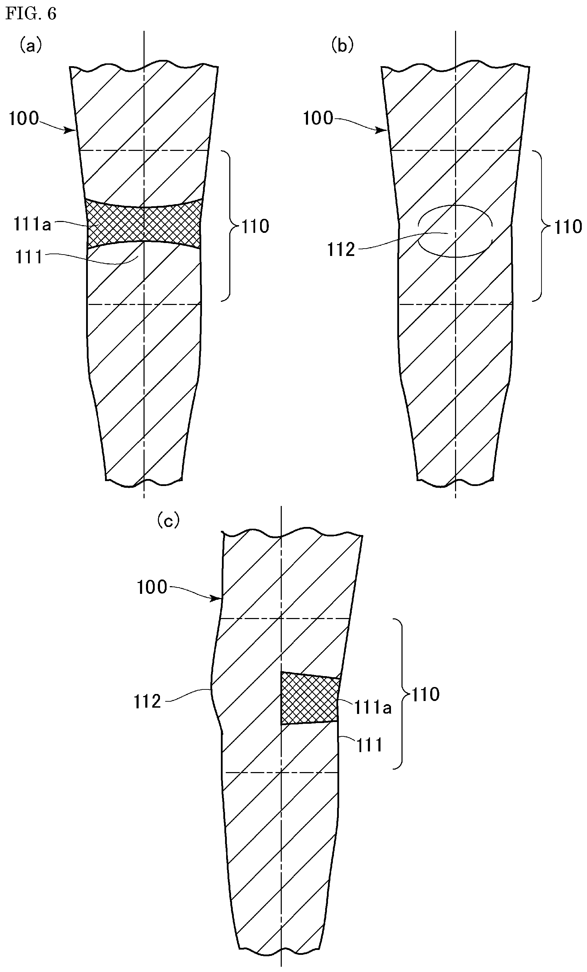

FIGs. 5 and FIGs. 6 are views for explaining an elastic stocking 100 as an elastic garment according to the second embodiment of the present invention. Note that the drawings mainly illustrate a tubular-shaped portion that covers a knee joint (a joint covering portion 110 (the range surrounded by the imaginary line centered on the knee)), of the elastic stocking 100 formed from the same knitted fabric as that in the first embodiment. The joint covering portion 110 of the elastic stocking 100 has an inner covering portion 111 that covers a flexion-direction inner side of the knee joint (back of the knee), and a knitted fabric part of the inner covering portion 111 at least partly has a course-reduced portion 111a having a smaller number of stitch courses in the wale direction than an outer covering portion 112 that covers a flexion-direction outer side of the knee joint located on the opposite side (see FIGs. 6(a), (c)).

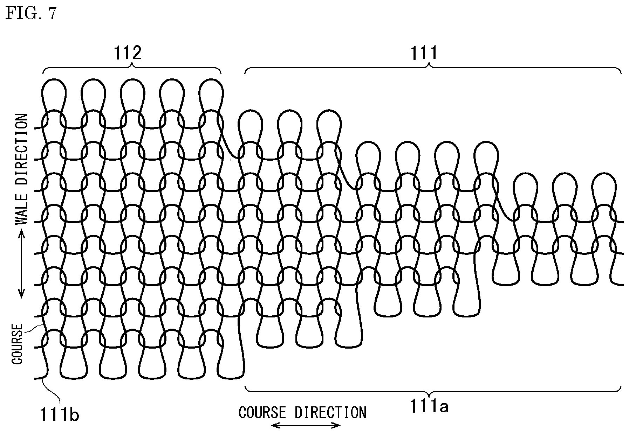

To knit the joint covering portion 110 of the elastic stocking 100, a yarn 111b is knitted in the course direction by a weft knitting machine while forming loops, as illustrated in FIG. 7. In a range of a knitted fabric constituting the outer covering portion 112 of the joint covering portion 110, knitting is performed without reducing the number of courses in the wale direction, but the knitting does not proceed further when reaching the range of the inner covering portion 111, but returns to the range of the outer covering portion 112 to stack courses in the wale direction and proceeds in the course direction. This is repeated, and when reaching a predetermined course, knitting is performed in the course direction up to the inner covering portion 111, and after the necessary number of courses is knitted in the inner covering portion 111, the knitting returns to the range of the outer covering portion 112. This is repeated, and when the knitting of the range of the joint covering portion 110 is completed, the knitting proceeds in the ankle direction or the thigh direction, whereby the elastic stocking 100 is formed. Although details are not illustrated in FIG. 7, the ground elastic yarn 21 and the thread elastic yarn 31 are used as the yarn 111b, and the plain knit structures 20 and the thread knit structures 30 are formed to alternate in the wale direction as illustrated in FIGs. 4(a), (b), as in the first embodiment. However, in this embodiment, the knitted fabric forming the inner covering portion 111 has the course-reduced portion 111a where the number of stitch courses in the wale direction is smaller than the number of stitch courses in the wale direction in the knitted fabric forming the outer covering portion 112.

Here, in the joint covering portion 110, the inner covering portion 111 and the outer covering portion 112 may be divided at approximately half positions in the circumferential direction of the joint covering portion 110 as illustrated in FIG. 6(c), but the ranges of the inner covering portion 111 and the outer covering portion 112 can be set such that, in the range around a human joint, parts of side surfaces of the flexion direction are excluded, and a range inward of these parts of the side surfaces is defined as the inner covering portion 111 and a range outward of these parts of the side surfaces is defined as the outer covering portion 112.

As described above, in the elastic stocking 100 of this embodiment, the knitted fabric constituting the inner covering portion 111 at least partly has the course-reduced portion 111a having a smaller number of courses in the wale direction than the outer covering portion 112. Since the elastic stocking 100 includes the elastic yarns, it is designed to exert a predetermined compression. When a foot is inserted to wear it while resisting that pressure, it stretches not only in the radial directions of the foot and the leg but also in the longitudinal directions thereof. At this time, the course-reduced portion 111a of the inner covering portion 111 stretches more in the longitudinal direction of the leg than the outer covering portion 112 to have a higher tension than the outer covering portion 112. As a result, when the knee is flexed, the inner covering portion 111, which has stretched more at the back of the knee, tends to contract quickly, making slack and wrinkles unlikely to occur at the back of the knee. This allows the inner covering portion 111 to apply a predetermined compression to the worn region. The occurrence of slack or wrinkles decreases the compression generated on that region. Furthermore, it is possible to prevent the slack or wrinkles from digging into the skin and obstructing lymph flow, blood flow, or the like. In addition, in this embodiment, the simple configuration of forming the course-reduced portion on the joint flexion-direction inner side enables to decrease slack and wrinkles. This is simpler than the configuration of Patent Document 2, which requires measuring the circumferential lengths of various regions including the joint, and can also reduce manufacturing costs.

This embodiment is configured to reduce the number of courses in the inner covering portion 111, but the course-direction range where the number of courses is reduced (the number of stitches) is not limited. The range may be the entire course-direction range of the knitted fabric constituting the inner covering portion 111, or it may be a course-direction part of the knitted fabric part. However, since the tubular-shaped joint covering portion 110 stretches in the longitudinal direction of the leg at the time of wearing, and the range with the reduced number of courses stretches more than the opposite outer covering portion 112. Consequently, its reaction force acts strongly, but depending on the number of courses reduced, there is a possibility of breakage due to overstretching. Therefore, it is preferable that the course-direction range where the number of courses is reduced be only a part. Moreover, in the elastic stocking 100 of this embodiment, the course-reduced portion 111a is provided in the joint covering portion 110 that covers the knee joint, but it can also be provided in a portion covering an ankle joint, a portion covering a hip joint, or the like, and furthermore, the course-reduced portions 111a can be provided in a plurality of joint covering portions 110.

As in the first embodiment, the form of the elastic stocking 100 is applicable to one having a welt portion, a leg portion, and a foot portion and reaching above the knee, foot covers (products with only a foot portion), socks (products having a top rubber portion, a leg portion, and a foot portion and reaching below the knee), tights (products with a panty portion, leg portions, and foot portions), and further to those with a structure having no foot portion, such as leggings and compression shorts. In this specification, the term "lower-body wear" may be any as long as it has a portion that covers at least one of toe joints, an ankle joint, and a knee joint, as the joint covering portion, and includes not only the abovementioned "socks" but also one with a structure having no foot portion.

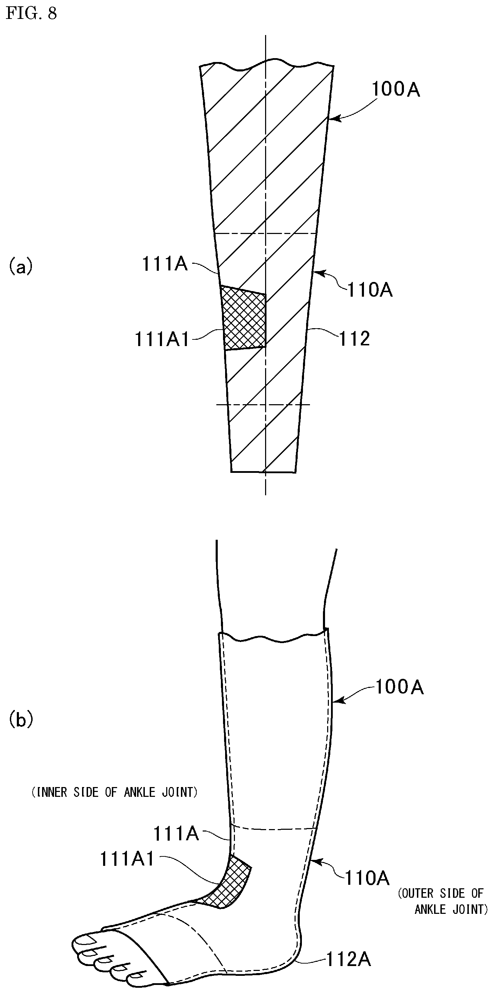

FIGs. 8(a), (b) illustrate an example of an elastic stocking 100A in which a course-reduced portion 111A1 is provided in a joint covering portion 110A for an ankle joint. In the case of a configuration covering the ankle joint, a typical stocking has a shape adapted to the heel which bulges outward, and a dedicated portion for covering the heel is formed. In the first embodiment, as illustrated in FIG. 1, the heel-corresponding knitted fabric 42 is also provided. In contrast, in the elastic stocking 100A illustrated in FIG. 8(a), the joint covering portion 110A covering an ankle joint is formed in a substantially tubular shape, and similar to the abovementioned joint covering portion 110 covering a knee joint, the course-reduced portion 111A1 having fewer courses than an outer covering portion 112A is provided in an inner covering portion 111A in terms of the flexion direction of the ankle joint. With this configuration, as illustrated in FIG. 8(b), a pulling force in the stretching direction acts relatively more strongly on the inner covering portion 111A covering the inner side of the ankle joint (inner side of the ankle) than on the outer covering portion 112A. Thus, even though a dedicated portion for covering the heel is not formed unlike in a typical stocking, the ankle joint can naturally maintain a posture bent at approximately 90 degrees, and the occurrence of slack and wrinkles in the inner covering portion 111A can also be reduced. Therefore, it is possible to inhibit a decrease in necessary compression on the inner side of the ankle, and also inhibit digging of slack or wrinkles, and the like. Furthermore, without a need to form a dedicated portion for covering the heel, knitting of the elastic stocking 100 near the ankle is facilitated, which can contribute to simplifying the knitting process and reducing manufacturing costs. Note that it is also possible to have a configuration in which the course-reduced portion 111A1 is provided in the inner covering portion 111A as in this embodiment while providing a dedicated portion for covering the heel (the heel-corresponding knitted fabric 42 of the first embodiment). This also applies to other joints such as a knee joint.

In addition, the configuration of this embodiment is applicable to any elastic garment as long as it has a tubular-shaped joint covering portion worn around a joint, and is applicable to a sleeve having a portion covering at least one of a wrist joint, an elbow joint, and a shoulder joint, a glove having, as the joint covering portion, a portion covering at least one of a wrist joint and finger joints, or upper-body wear such as a garment in which a sleeve or a glove is integrally provided. It is also applicable to full-body wear such as a bodysuit in which lower-body wear and upper-body wear are integrated.

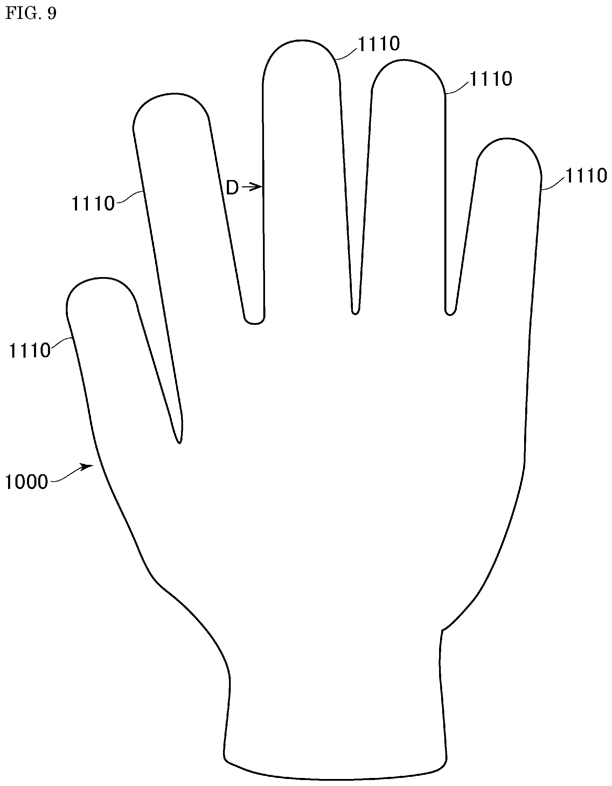

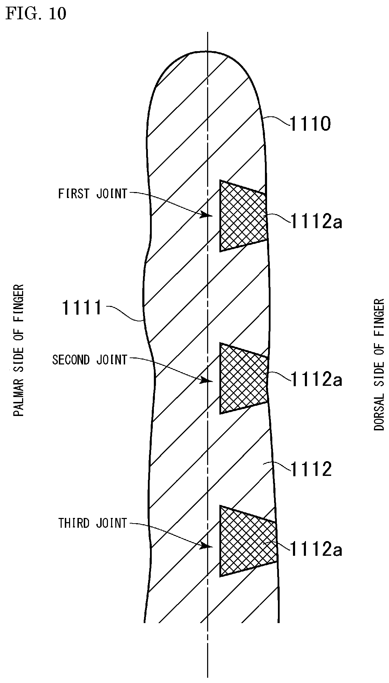

FIG. 9 and FIG. 10 illustrate the third embodiment of the present invention. In this embodiment, a glove 1000 is shown as the elastic garment. In the glove 1000, joint covering portions 1110 covering fingers are formed in a tubular shape, and in the joint covering portions 1110, in at least part of knitted fabric parts of outer covering portions 1112 covering flexion-direction outer sides of the finger joints (dorsal sides of the fingers), the number of stitch courses in the wale direction is smaller than in inner covering portions 1111, which are located on the opposite side, covering the finger palmar sides, which are on the inner sides in terms of the flexion direction of the joints.

As illustrated in FIG. 10, for example, in a portion where to insert the middle finger, course-reduced portions 1112a are formed in the outer covering portion 1112 in ranges corresponding to the first, second, and third joints. A method of reducing the number of courses in the outer covering portions 1112 for these finger joints is exactly the same as the method of knitting the inner covering portion 111 located on the flexion-direction inner side of the knee joint, in the elastic stocking 100 of the second embodiment illustrated in FIG. 7.

According to this embodiment, the course-reduced portions 1112a are provided in the outer covering portions 1112 for the finger joints. Therefore, when the glove 1000 is worn, contrary to the above-described embodiments, the contractive force of the elastic yarn in the course-reduced portions 1112a of the outer covering portions 1112 acts more strongly than in the inner covering portions 1111. Accordingly, a force always acts on this glove 1000 in the direction of the outer covering portions 1112 of the fingers, that is, in a direction of warping back, and when the glove 1000 is worn, this force in the warping direction acts as a reaction force, and the reaction force becomes resistance at the time of an attempt to bend the fingers in the flexion direction. Consequently, by performing an act of gripping the hand with the glove 1000 worn, it is possible to build and improve muscle strength.

In this embodiment as well, it is possible to appropriately adjust the reaction force that acts as resistance at the time of the bending in the flexion direction, by adjusting the ranges of the knitted fabric parts with fewer courses or adjusting the amount of the elastic yarn used, its usage rate, the type of the elastic yarn, or the like in the outer covering portions 1112.

Furthermore, the elastic garment for improving muscle strength by reducing the number of courses in the knitted fabrics forming the outer covering portions 1112 in the joint covering portions 1110 as in this embodiment is not limited to the glove 1000. As in the second embodiment, it is also applicable to lower-body wear, such as socks covering the lower limbs, sleeves worn on the arms, and the like.

Further, the second embodiment describes the elastic stocking 100 in which the number of courses in the inner covering portion 111 is reduced. In the case where the elastic stocking 100 is formed to have a simple tubular shape without a toe portion, it can be worn back-to-front, that is, worn such that the position of the inner covering portion 111 where the number of courses is reduced is on the outer side of the knee joint, resulting in a configuration in which the course-reduced portion is provided in the outer covering portion as in the third embodiment. As a result, resistance is applied when the knee is bent, allowing it to be used for strengthening and improving the muscle strength of the leg.

In the case of the glove 1000 of the third embodiment in which the number of courses in the outer covering portions 1112 is reduced, since it is usually adapted to the shapes of the five fingers, wearing it back-to-front would cause discomfort. Therefore, the left and right hands are swapped for wear. Consequently, the course-reduced portions 1112a formed in the outer covering portions 1112 are positioned in the inner covering portions on the opposite hand. Thus, as in the first embodiment, a configuration capable of inhibiting slack and wrinkles during flexion and inhibiting their digging into the skin can be achieved.

In this way, the elastic garment of the present invention by itself can exhibit different functions, by being worn back-to-front or by being worn with the left and right hands swapped. Designing the shape, decorative patterns, and the like to be compatible with these functions enables a common structure for the improvement of lymphedema and the like and for the improvement of muscle strength, which is economical.

### Explanation of Reference Signs

- 1: elastic stocking
- 10: knitted fabric
- 10a: stepped surface
- 10b: flat surface
- 20: plain knit structure
- 21: ground elastic yarn
- 30: thread knit structure
- 31: thread elastic yarn
- 41: knee-corresponding knitted fabric
- 42: heel-corresponding knitted fabric
- 100: elastic stocking
- 110: joint covering portion
- 111: inner covering portion
- 111a: course-reduced portion
- 111b: yarn
- 112: outer covering portion
- 1000: glove
- 1110: joint covering portion
- 1111: inner covering portion
- 1112: outer covering portion
- 1112a: course-reduced portion

## Claims

1. An elastic garment that is knitted using at least two types of elastic yarns different in stretchability, the elastic garment being formed from a knitted fabric in which a plain knit structure and a thread knit structure are alternately knitted,
the plain knit structure being knitted using a ground elastic yarn, and
the thread knit structure being knitted using the ground elastic yarn and a thread elastic yarn that is thicker and higher in stretch modulus than the ground elastic yarn.

2. The elastic garment according to claim 1,
wherein the ground elastic yarn and the thread elastic yarn which constitute the thread knit structure are knitted such that knit and miss positions of the ground elastic yarn and the thread elastic yarn alternate stitch by stitch in a course direction.

3. The elastic garment according to claim 1,
wherein the plain knit structure and the thread knit structure are knitted to alternate in a wale direction.

4. The elastic garment according to claim 1,
wherein core yarns of both the ground elastic yarn and the thread elastic yarn are polyurethane yarns, and
wherein the polyurethane yarn constituting the ground elastic yarn has a thickness of 150 decitex or less, and the polyurethane yarn constituting the thread elastic yarn has a thickness of 550 decitex or more.

5. The elastic garment according to claim 4,
wherein the thickness of the polyurethane yarn constituting the ground elastic yarn is within a range of 30 to 100 decitex, and the thickness of the polyurethane yarn constituting the thread elastic yarn is within a range of 1000 to 2500 decitex.

6. The elastic garment according to claim 1, wherein the knitted fabric is composed of only either a knit structure or a purl structure.

7. The elastic garment according to claim 1,
the elastic garment being intended for improving or preventing lymphedema or venous return disorder.

8. The elastic garment according to claim 1, comprising
a tubular-shaped joint covering portion having an inner covering portion that covers a flexion-direction inner side of a joint and an outer covering portion that covers a flexion-direction outer side of the joint,
wherein a knitted fabric part of one of the inner covering portion and the outer covering portion in the joint covering portion at least partly has a course-reduced portion that has a smaller number of stitch courses in a wale direction than a knitted fabric part forming the other.

9. The elastic garment according to claim 8,
wherein the course-reduced portion is provided in at least part of the knitted fabric part of the inner covering portion.

10. The elastic garment according to claim 9,
the elastic garment being intended to improve or prevent lymphedema or venous return disorder.

11. The elastic garment according to claim 8,
wherein the course-reduced portion is provided in at least part of the knitted fabric part of the outer covering portion.

12. The elastic garment according to claim 11,
the elastic garment being intended to improve muscle strength.

13. The elastic garment according to claim 8, the elastic garment being:
lower-body wear that has, as the joint covering portion, a portion covering at least one of a toe joint, an ankle joint, and a knee joint;
upper-body wear that has, as the joint covering portion, a portion covering at least one of a wrist joint, an elbow joint, a shoulder joint, and a finger joint; or
full-body wear in which the lower-body wear and the upper-body wear are integrated.

14. A method for producing an elastic garment that is knitted using an elastic yarn and includes a tubular-shaped joint covering portion having: an inner covering portion covering a flexion-direction inner side of a joint; and an outer covering portion covering a flexion-direction outer side of the joint, the method comprising,
when knitting the joint covering portion,
making the number of stitch courses in a wale direction in at least part of a knitted fabric part of one of the inner covering portion and the outer covering portion smaller than in a knitted fabric part forming the other.

15. The method for producing the elastic garment according to claim 14,
the method being a method for producing an elastic garment for improving or preventing lymphedema or venous return disorder, by making the number of courses in at least part of the knitted fabric part of the inner covering portion smaller than in the knitted fabric part forming the outer covering portion.

16. The method for producing the elastic garment according to claim 14,
the method being a method for producing an elastic garment for improving muscle strength, by making the number of courses in at least part of the knitted fabric part of the outer covering portion smaller than in the knitted fabric part forming the inner covering portion.
